# EUROPÄISCHE PATENTSCHRIFT

(11) **EP 2 349 112 B1**
(45) Veröffentlichungstag und Bekanntmachung des Hinweises auf die Patenterteilung: **23.07.2014**
(21) Anmeldenummer: 09736553.0
(22) Anmeldetag: 17.06.2009
(51) Int. Cl.: A61F 2/44

(54) **IMPLANTAT**
IMPLANT
IMPLANT

(43) Veröffentlichungstag der Anmeldung: 03.08.2011
(73) Patentinhaber: Ulrich GmbH & Co. KG, 89081 Ulm (DE)
(72) Erfinder: GERNER, Leonie, 89081 Ulm (DE)
(74) Vertreter: Hentrich, Swen
(86) Internationale Anmeldenummer: PCT/DE2009/075026
(87) Internationale Veröffentlichungsnummer: WO 2010/145627

(56) Entgegenhaltungen:
- EP-A1- 1 398 008
- WO-A1-2004/100837
- WO-A1-2005/000170
- DE-A1- 4 109 941
- DE-U1- 20 307 876
- US-A1- 2007 255 421
- US-B2- 6 808 538

## Beschreibung

Die Erfindung betrifft ein Implantat zum Einsetzen zwischen Wirbelkörper der Wirbelsäule, mit einem als Hülse gestalteten Implantatteil, das an mindestens einem seiner freien Enden eine Endplatte aufweist, der eine mit dem Hohlraum der Hülse kommunizierende Durchgangsöffnung zugeordnet ist.

Derartige Implantate werden in der Praxis eingesetzt, um z.B. bei degenerativen oder traumatischen Erkrankungen der Wirbelsäule die Stabilität wieder herstellen zu können, wobei das Implantatteil beispielsweise als Platzhalter Verwendung findet, um den Abstand zwischen benachbarten Wirbelkörpern, der aufgrund der vollständigen oder teilweisen Entfernung eines erkrankten Wirbelkörpers dessen Längserstreckung aufweisen kann, zu überbrücken. In der Praxis finden derartige Implantate auch Verwendung als Ersatz für eine entfernte Bandscheibe. Die Endplatte dient dazu, die Flächenpressung zu reduzieren. Als nachteilig bei derartigen Implantaten ist beurteilt worden, dass mit derartigen Implantaten keine Anpassung an die natürliche Krümmung der Wirbelsäule möglich ist, so dass im Stand der Technik weiterhin vorgeschlagen worden ist, zwischen dem Implantatteil und der Endplatte eine scharnierartige Verbindung vorzusehen, bei der die Endplatte um eine Drehachse relativ zu dem Implantatteil verschwenkt werden kann. Beispielhaft kann dies bezüglich auf die US 6 808 538 B2 verwiesen werden, die ein Implantat mit einem Implantatteil zeigt, das an seinen freien Enden jeweils zwei gegenüberliegende Zapfen besitzt, von denen ausgehend die Seitenränder des Implantats geneigt abfallen. Auf diese Zapfen kann die Endplatte aufgeklipst werden und in dem von der Neigung der Seitenränder vorgegebenen Winkelbereich verschwenkt werden.

Der Erfindung liegt die Aufgabe zugrunde, ein Implant der eingangs genannten Art so auszubilden, dass eine verbesserte Anpassungsmöglichkeit an die individuellen Gegebenheiten des Patienten möglich ist.

Diese Aufgabe wird bei einem Implantat der eingangs genannten Art dadurch gelöst, dass in der Endplatte auf der dem Implantatteil zuweisenden Seite eine rotationssymmetrische Ausnehmung ausgebildet ist, die durch ihre Seitenwand an einem durch eine Ringnut begrenzten Ringbund des Implantatteils geführt ist, und dass die Endplatte mit einem vom Außenumfang bis zur Durchgangsöffnung reichenden Schlitz versehen ist und die dadurch gebildeten Schenkel der ringförmigen Endplatte lösbar miteinander verbindbar sind.

Mit dieser Ausführungsform ist der Vorteil verbunden, dass zum einen ein polyaxiales Gelenk bereitgestellt ist, bei dem die Schwenkmöglichkeit nicht nur auf eine Schwenkachse begrenzt ist, sondern, in Abhängigkeit der konkreten Implantationsstelle sowie der individuellen Krümmung der Wirbelsäule des Patienten, ein Verdrehen der Endplatte relativ zu dem Implantatteil infolge der Rotationssymmetrie der Ausnehmung um unterschiedliche Drehachsen erfolgen kann, wobei eine selbstständige Anpassung des Winkels zwischen der Endplatte und dem Implantatteil im sagittal und frontalen Profil während der Implantation erfolgt. Zu beachten ist weiterhin, dass die Rotationssymmetrie der Ausnehmung auch ermöglicht, dass eine Verdrehung um die Längsachse des Implantatteils der Endplatte erfolgt da weiterhin auf Bauteile verzichtet wird, die eine entsprechende Drehung behindern, wie dies noch durch die Zapfen in der US 6 808 538 B2 erfolgt. Durch die Anbringung eines vom Außenumfang zur Durchgangsöffnung reichenden Schlitz an der Endplatte können die dadurch gebildeten Schenkel der ringförmigen Endplatte lösbar miteinander verbindbar sein, beispielsweise durch eine Schraubverbindung, was die Verbindung des Implantatteils mit der Endplatte zur Montage des Implantats vereinfacht.

Weitere mit dem erfindungsgemäßen Implantat verbundene Vorteile sind dadurch gegeben, dass das dem Operateur zur Verfügung zu stellende Implantatsystem aus weniger Komponenten bestehen kann, da mehrere Winkel mit einem (vormontierten) Implantat abgedeckt werden können, so dass durch das Implantatsystem kleineren Umfangs sich auch die Lagerhaltungskosten für die Krankenhäuser sowie den Hersteller reduzieren.

Zu beachten ist weiterhin, dass bei dem erfindungsgemäßen Implantat die Lagerung der Endplatte an dem Implantatteil im wesentlichen seitlich erfolgt, da die Endplatte mit der Ausnehmung den Ringbund des Implantatteils seitlich übergreift, wobei die Ringnut dazu dient, das Verschwenken der Endplatte gegenüber dem Implantatteil zu ermöglichen.

In diesem Zusammenhang ist es bevorzugt, wenn die Seitenwand im Querschnitt konkav gekrümmt ist und der Ringbund einen in Längsrichtung des Implantatteils gerundeten Außenumfang besitzt, um so eine stetige Bewegung der Endplatte gegenüber dem Implantatteil zu ermöglichen.

Nochmals bevorzugt in diesem Zusammenhang ist dabei, wenn die einander zur Anlage bestimmten Oberflächen der Ausnehmung sowie des Ringbundes Abschnitten einer Hohlkugel bzw. Kugel entsprechen, da die Eigenschaften eines Kugelgelenkes mit einem Kugelkopf und einer Kugellagerschale nachgebildet werden und bei einer entsprechend weiten Umfassung des Ringbundes durch die Seitenwand der Ausnehmung die Lagesicherung des Ringbundes in der Ausnehmung erzielt werden kann.

Wenn allerdings auf eine entsprechend weiträumige Umgreifung verzichtet wird oder der Ringbund nur eine im Vergleich zur Tiefe der Ausnehmung begrenzte Dicke aufweisen soll, um einen zusätzlichen Bewegungsfreiheitsgrad zu generieren, bietet es sich an, dass die Ausnehmung auf der zum Implantatteil weisenden Seite einen die Öffnungsweite verengenden, zum Eingreifen in die Ringnut bestimmten Ausnehmungsbund aufweist, der die Lagesicherung gewährleistet.

Vorteilhaft ist es weiterhin, wenn die Breite der Ringnut im wesentlichen der Tiefe der Ausnehmung entspricht, da so nicht die Seitenwände der Ringnut die Verschwenkmöglichkeit der Endplatte gegenüber dem Implantatteil begrenzen.

Eine im Rahmen der Erfindung wiederum ganz besonders bevorzugte Ausführungsform der Erfindung ist dadurch gekennzeichnet, dass in der Wand der Hülse Öffnungen ausgebildet sind. Mit dieser Gestalt wird ein großer, offener Innenraum zur Verfügung gestellt, der mit autologem oder homologem Knochenmaterial oder anderen Füllmaterialien aufgefüllt werden kann, so dass dadurch das Einwachsen des Implantates gefördert wird.

Eine alternative Möglichkeit der Verbindung besteht darin, dass der Ausnehmungsbund und gegebenenfalls die Oberfläche des Ringbundes als Gewindegang ausgebildet ist, also der Ausnehmungsbund so über den Ringbund geschraubt werden kann, dass der Ausnehmungsbund in die Ringnut eintritt und damit dann die freie Drehbarkeit des polyaxialen Gelenkes erreicht ist.

Im folgenden wird die Erfindung an in der Zeichnung dargestellten Ausführungsbeispielen näher erläutert; es zeigen:
- Fig. 1: einen Längsschnitt durch ein erfindungsgemäßes Implantat mit der gegenüber dem Implantatteil im Winkel 0° ausgerichteten Endplatte,
- Fig. 2: eine der Figur 1 entsprechenden Darstellung mit der Winkelstellung der Endplatte gegenüber dem Implantatteil, und
- Fig. 3: eine perspektivische Darstellung eines Implantats mit einer die Durchgangsöffnung sowie eine Schraubverbindung aufweisenden Endplatte, und
- Fig. 4: einen Längsschnitt eines Implantats mit zwei Endplatten.

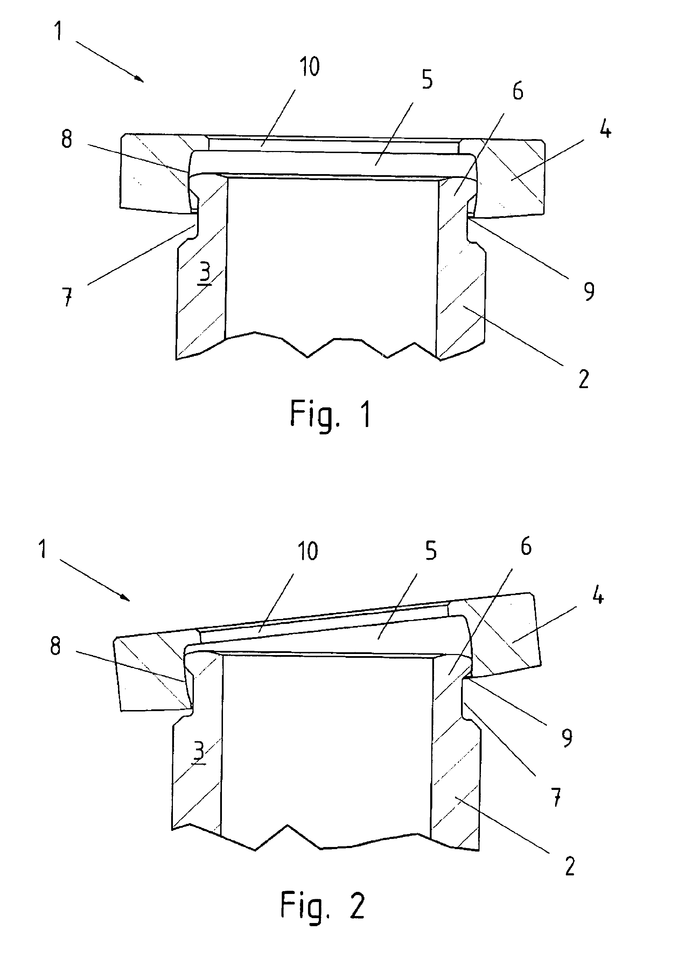

In der Zeichnung in den Figuren 1 bis 3 sind zwei Ausführungsformen eines Implantates 1 gezeigt, das zum Einsetzen zwischen Wirbelkörper der Wirbelsäule dient als Ersatz von teilweise oder vollständig entfernten Wirbeln. Dieses Implantat 1 weist ein Implantatteil 2 auf, das als Hülse 3 gestaltet ist, wobei Öffnungen in der Wand der Hülse 3 ausgebildet sein können. An mindestens einem der freien Enden des Implantatteils 2, vorzugsweise aber an beiden freien Enden ist jeweils eine Endplatte 4 angeordnet, die auf der dem Implantatteil 2 zuweisenden Seite eine rotationssymmetrische Ausnehmung 5 aufweist. An den freien Enden des Implantatteils 2 ist ein Ringbund 6 ausgebildet, der auf der Innenseite durch eine Ringnut 7 begrenzt ist, wobei dieser Ringbund 6 zur Führung der Endplatte 4 über die Seitenwand 8 der Ausnehmung 5 dient. Dazu ist die Seitenwand 8 im Querschnitt konkav gekrümmt und der Ringbund 6 besitzt einen gerundeten Außenumfang, wobei in dem gezeigten Ausführungsbeispiel die Oberflächen der Ausnehmung 5 sowie des Ringbundes Abschnitten einer Hohlkugel bzw. Kugel entsprechen.

Bei dem in der Zeichnung dargestellten Ausführungsbeispiel besitzt die Ausnehmung 5 weiterhin auf der zum Implantatteil 2 weisenden Seite einen die Öffnungsweite verengenden, zum Eingreifen in die Ringnut bestimmten Ausnehmungsbund 9, der auch als Gewindegang ausgebildet sein kann, um im Zusammenwirken mit einem korrespondierenden Gewindegang des Ringbundes 6 ein Aufschrauben der Endplatte 4 auf das Implantatteil 2 zu ermöglichen, bis nach dem Austreten des endplattenseitigen Gewindeganges in die Ringnut 7 die Bewegungsfreiheitsgrade der Endplatte 4 gegenüber dem Implantatteil 2 wieder gegeben sind, nämlich zum einen eine Verdrehbarkeit in die Längsachse des Implantatteils 2 sowie eine polyaxiale Verschwenkbarkeit in Abhängigkeit von den sich während der Implantation konkret zeigenden Randbedingungen.

Die Figuren 1 und 2 lassen erkennen, dass die Breite der Ringnut 7 im wesentlichen der Tiefe der Ausnehmung 5 entspricht.

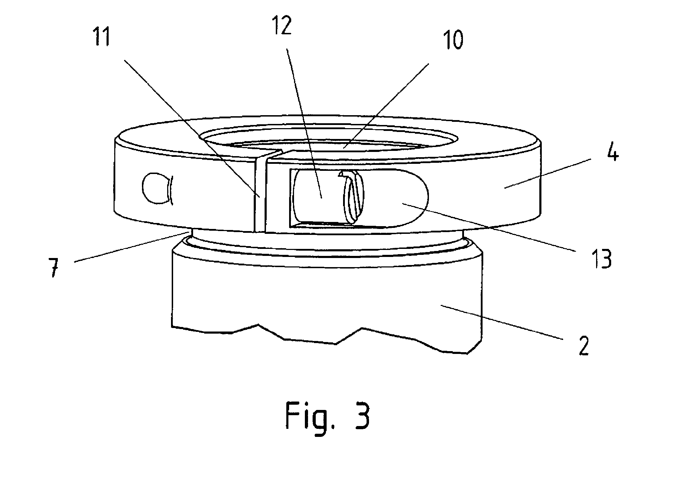

Figur 3 zeigt eine Ausführungsform, bei der die Endplatte 4 mit einem vom Außenumfang bis zu einer Durchgangsöffnung 10 reichenden Schlitz 11 versehen ist und die dadurch gebildeten Schenkel der ringförmigen Endplatte 4 lösbar miteinander verbunden sind, wobei die Verbindung durch eine Verbindungsschraube 12 erfolgt, die in ein dem einem Schenkel zugeordnetes Gewinde eingreift. Der Schraubenkopf ist dabei in einer Senkbohrung 13 versenkt.

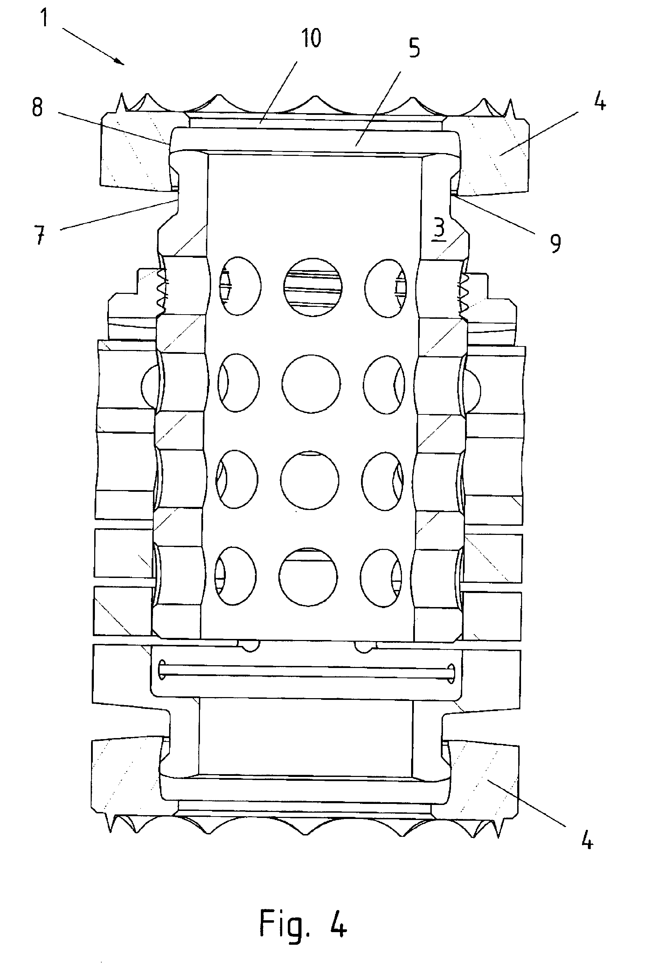

Figur 4 zeigt ein vollständiges Implantat 1 mit zwei an den gegenüberliegenden Enden angeordneten Endplatten 4, die jeweils gegenüber dem zugeordneten, zur Anlage kommenden Wirbelkörper die geeignete Lage einnehmen können, und zwar auch mit von einander abweichenden Winkeln. Die Hülse 3 weist in ihrem Umfang eine Mehrzahl von Öffnungen auf, die ein Befüllen und/oder ein verbessertes Einwachsen des Implantats 1 ermöglichen.

### Bezugszeichenliste

- 1: Implantat
- 2: Implantatteil
- 3: Hülse
- 4: Endplatte
- 5: Ausnehmung
- 6: Ringbund
- 7: Ringnut
- 8: Seitenwand
- 9: Ausnehmungsbund
- 10: Durchgangsöffnung
- 11: Schlitz
- 12: Verbindungsschraube
- 13: Senkbohrung

## Patentansprüche

1. Implantat zum Einsetzen zwischen Wirbelkörper der Wirbelsäule, mit einem als Hülse (3) gestalteten Implantatteil (2), das an mindestens einem seiner freien Enden eine Endplatte (4) aufweist, der eine mit dem Hohlraum der Hülse (3) kommunizierende Durchgangsöffnung (10) zugeordnet ist, wobei in der Endplatte (4) auf der dem Implantatteil (2) zuweisenden Seite eine rotationssymmetrische Ausnehmung (5) ausgebildet ist, die durch ihre Seitenwand (8) an einem durch eine Ringnut (7) begrenzten Ringbund (6) des Implantatteils (2) geführt ist, **dadurch gekennzeichnet, dass** die Endplatte (4) mit einem vom Außenumfang bis zur Durchgangsöffnung reichenden Schlitz (11) versehen ist und die dadurch gebildeten Schenkel der ringförmigen Endplatte (4) lösbar miteinander verbindbar sind.

2. Implantat nach Anspruch 1, **dadurch gekennzeichnet, dass** die Seitenwand (8) im Querschnitt konkav gekrümmt ist und der Ringbund (6) einen in Längsrichtung des Implantatteils (2) gerundeten Außenumfang besitzt.

3. Implantat nach Anspruch 2, **dadurch gekennzeichnet, dass** die einander zur Anlage bestimmten Oberflächen der Ausnehmung (5) sowie des Ringbundes (6) Abschnitten einer Hohlkugel bzw Kugel entsprechen.

4. Implantat nach Anspruch 2 oder 3, **dadurch gekennzeichnet, dass** die Ausnehmung (5) auf der zum Implantatteil (2) weisenden Seite einen die Öffnungsweite verengenden, zum Eingreifen in die Ringnut (7) bestimmten Ausnehmungsbund (9) aufweist.

5. Implantat nach einem der Ansprüche 1 bis 4, **dadurch gekennzeichnet, dass** die Breite der Ringnut (7) im wesentlichen der Tiefe der Ausnehmung (5) entspricht.

6. Implantat nach Anspruch 5, **dadurch gekennzeichnet, dass** in der Wand der Hülse (3) Öffnungen ausgebildet sind.

7. Implantat nach einem der Ansprüche 4 bis 6, **dadurch gekennzeichnet, dass** der Ausnehmungsbund (9) und gegebenenfalls die Oberfläche des Ringbundes (6) als Gewindegang ausgebildet ist.

## Claims

1. An implant for insertion between vertebrae of the spinal column, comprising an implant portion (2) which is in the form of a sleeve (3) and which at at least one of its free ends has an end plate (4) with which there is associated a through opening (10) communicating with the hollow space in the sleeve (3), wherein provided in the end plate (4) on the side facing towards the implant portion (2) is a rotationally symmetrical recess (5) which is guided by its side wall (8) at an annular collar (6) of the implant portion (2), that is defined by an annular groove (7), **characterised in that** the end plate (4) is provided with a slot (11) extending from the outside periphery to the through opening and the limbs formed thereby of the annular end plate (4) can be releasably connected together.

2. An implant according to claim 1 **characterised in that** the side wall (8) is concavely curved in cross-section and the annular collar (6) has an outside periphery rounded in the longitudinal direction of the implant portion (2).

3. An implant according to claim 2 **characterised in that** the surfaces which are intended to bear against each other of the recess (5) and the annular collar (6) correspond to portions of a hollow sphere or a sphere.

4. An implant according to claim 2 or claim 3 **characterised in that** on the side facing towards the implant portion (2) the recess (5) has a recess collar (9) which reduces the width of the opening and which is intended to engage into the annular groove (7).

5. An implant according to one of claims 1 to 4 **characterised in that** the width of the annular groove (7) substantially corresponds to the depth of the recess (5).

6. An implant according to claim 5 **characterised in that** openings are provided in the wall of the sleeve (3).

7. An implant according to one of claims 4 to 6 **characterised in that** the recess collar (9) and optionally the surface of the annular collar (6) is in the form of a thread flight.

## Revendications

1. Implant destiné à être inséré entre des corps vertébraux du rachis, comprenant un élément d'implant (2) conformé en manchon (3), qui présente, à au moins une de ses extrémités libres, une plaque d'extrémité (4) à laquelle est associée une ouverture de passage (10) communiquant avec la cavité du manchon (3), la plaque d'extrémité (4) comportant, sur la face tournée vers l'élément d'implant (2), un évidement (5) symétrique de révolution qui est guidé par sa paroi latérale (8) sur un collet annulaire (6), délimité par une rainure annulaire (7), de l'élément d'implant (2), **caractérisé en ce que** la plaque d'extrémité (4) est pourvue d'une fente (11), s'étendant de la périphérie extérieure jusqu'à l'ouverture de passage, et les ailes ainsi formées de la plaque d'extrémité (4) annulaire peuvent être reliées de façon séparable l'une à l'autre.

2. Implant selon la revendication 1, **caractérisé en ce que** la paroi latérale (8) est incurvée en section transversale avec une forme concave et le collet annulaire (6) présente une périphérie extérieure qui est arrondie dans le sens de la longueur de l'élément d'implant (2).

3. Implant selon la revendication 2, **caractérisé en ce que** les surfaces de l'évidement (5) et du collet annulaire (6) qui sont destinées à être appliquées l'une contre l'autre correspondent à des portions d'une sphère creuse ou d'une sphère.

4. Implant selon la revendication 2 ou 3, **caractérisé en ce que** l'évidement (5) présente, sur le côté tourné vers l'élément d'implant (2), un rebord d'évidement (9) qui rétrécit la largeur d'ouverture et est destiné à s'engager dans la rainure annulaire (7).

5. Implant selon l'une des revendications 1 à 4, **caractérisé en ce que** la largeur de la rainure annulaire (7) correspond sensiblement à la profondeur de l'évidement (5).

6. Implant selon la revendication 5, **caractérisé en ce que** des ouvertures sont formées dans la paroi du manchon (3).

7. Implant selon l'une des revendications 4 à 6, **caractérisé en ce que** le rebord d'évidement (9) et, le cas échéant, la surface du collet annulaire (6) sont réalisés sous forme de filet.
